# EUROPÄISCHE PATENTSCHRIFT

(11) **EP 2 015 684 B1**
(45) Veröffentlichungstag und Bekanntmachung des Hinweises auf die Patenterteilung: **02.02.2011**
(21) Anmeldenummer: 07722203.2
(22) Anmeldetag: 13.04.2007
(51) Int. Cl.: A61B 17/16, A61C 8/00, A61B 19/00, A61B 17/00, A61C 3/02

(54) **BOHRER ZUR HERSTELLUNG VON BOHRUNGEN IM KNOCHENGEWEBE MIT EINEM HALTETEIL UND EINEM BOHRAUFSATZ**
TREPHINE FOR THE CREATION OF A BOREHOLE IN BONE TISSUE, WITH A HOLDING PART AND A DRILL BIT
FORET DESTINÉ À RÉALISER DES TROUS DANS LE TISSU OSSEUX AVEC PIÈCE DE RETENUE ET MÈCHE

(30) Priorität: 10.05.2006 DE 102006021748
(43) Veröffentlichungstag der Anmeldung: 21.01.2009
(73) Patentinhaber: Kirsch, Axel, Dr., 70794 Filderstadt (DE)
(72) Erfinder: DÜRR, Walter, 75196 Remchingen (DE); KIRSCH, Axel, 70794 Filderstadt (DE)
(74) Vertreter: Goddar, Heinz J.
(86) Internationale Anmeldenummer: PCT/DE2007/000645
(87) Internationale Veröffentlichungsnummer: WO 2007/128254

(56) Entgegenhaltungen:
- EP-A- 1 488 747
- EP-A- 1 582 171
- CH-A5- 694 133
- DE-A1-102005 011 917
- US-A- 3 597 582
- US-A- 4 951 690

## Beschreibung

Die Erfindung betrifft einen Bohrer zur Herstellung einer Bohrung in einem Knochengewebe nach der Einleitung des Patentanspruches 1.

Bei einem Bohrer dieser Art, wie er aus der DE 198 03 998 C2 bekannt ist, erfolgt die lösbare Verbindung der beiden Bohrerteile, d.h. des Halteteils und des Bohraufsatzes, im wesentlichen durch als Federzungen ausgebildete Rastelemente, die mit Aussparungen in dem jeweiligen anderen Teil in Eingriff bringbar sind. Bei dem bekannten Bohrer muß darauf geachtet werden, daß vor Beginn des Bohrvorganges der Bohraufsatz mit dem Halteteil durch entsprechende manuelle Verriegelung zuverlässig verbunden ist, da nur dann eine Festlegung des Bohraufsatzes, relativ zum Halteteil, sowohl in Axial- als auch in Drehrichtung gewährleistet ist. Ferner kann der Bohraufsatz des gattungsgemäßen Bohrers durchaus mehrfach verwendet werden, indem nämlich der Bohraufsatz nach Beendigung des Bohrvorganges vom Halteteil abgenommen und alsdann sterilisiert wird, wobei diese Sterilisation üblicherweise durch Erhitzung auf Temperaturen von 100 - 120°C erfolgt.

Die Notwendigkeit der manuell zu bewerkstelligenden zuverlässigen Verbindung zwischen Halteteil und Bohraufsatz einerseits und die mehrmalige Verwendungsmöglichkeit des Bohraufsatzes andererseits stellen an den Zahnarzt hohe Sorgfaltsanforderungen, einerseits bedingt durch die verhältnismäßig schwierige Manipulation des Bohraufsatzes und des Halteteiles und zum anderen durch die Notwendigkeit, aus hygienischen Gründen zuverlässig, nämlich durch geeignete Handhabungssorgfalt, zu gewährleisten, daß der Bohraufsatz nicht sterilisiert und dann erneut verwendet wird.

Der Erfindung liegt die Aufgabe zugrunde, den Bohrer nach der Einleitung des Patentanspruches 1 dahingehend weiterzubilden, dass eine mehrmalige Verwendung des Bohraufsatzes ausgeschlossen wird. Vorzugsweise soll dabei auch die Verriegelung zwischen Halteteil und Bohraufsatz vereinfacht werden.

Erfindungsgemäß wird diese Aufgabe durch die Merkmalskombination des Patentanspruches 1 gelöst.

Bevorzugte Ausführungsformen der Erfindung sind Gegenstand der Unteransprüche.

Weitere Merkmale und Vorteile der Erfindung ergeben sich aus der nachstehenden Beschreibung, in der ein Ausführungsbeispiel anhand der schematischen Zeichnung im einzelnen erläutert ist.

Dabei zeigt:
- Fig. 1: eine perspektivische Gesamtansicht eines Ausführungsbeispieles des Boh- rers nach der Erfindung, teilweise in Explosionsdarstellung;
- Fig. 2: den Bohrer von Figur 1 in der Seitenansicht, teilweise in Explosionsdarstel- lung;
- Fig. 3: einen Schnitt entlang der Linie III - III von Figur 2;
- Fig. 4: einen Bohraufsatz des Bohrers gemäß Fig. 1 - 3 in der Seitenansicht;
- Fig. 5: einen Schnitt entlang der Linie V - V von Figur 4;
- Fig. 6: ein Halteteil des Bohrers gemäß Fig. 1 - 3 in der Seitenansicht;
- Fig. 7: das Halteteil von Figur 6 in der Draufsicht (in Figur 6 von oben);
- Fig. 8.: einen Hülseneinsatz des Bohraufsatzes des Bohrers gemäß Fig. 1 - 5 in der Seitenansicht; und
- Fig. 9: einen Schnitt entlang der Linie IX - IX von Figur 8.

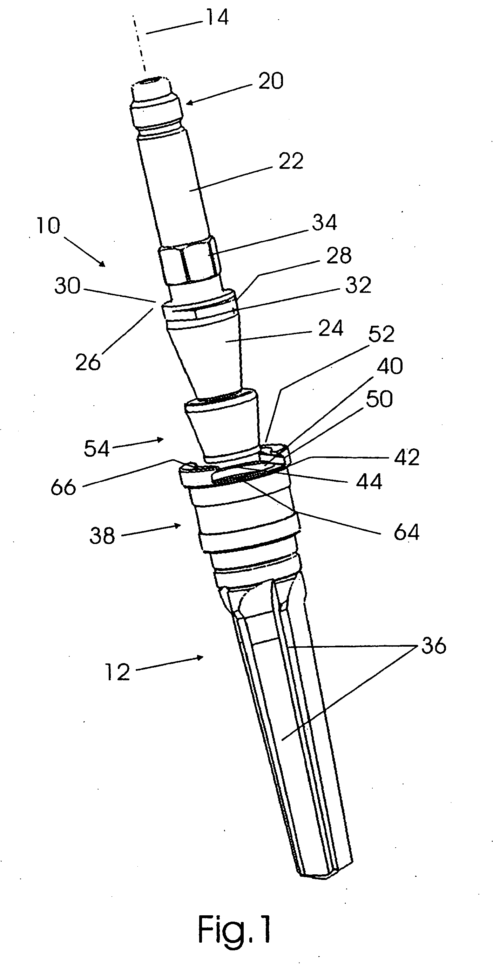

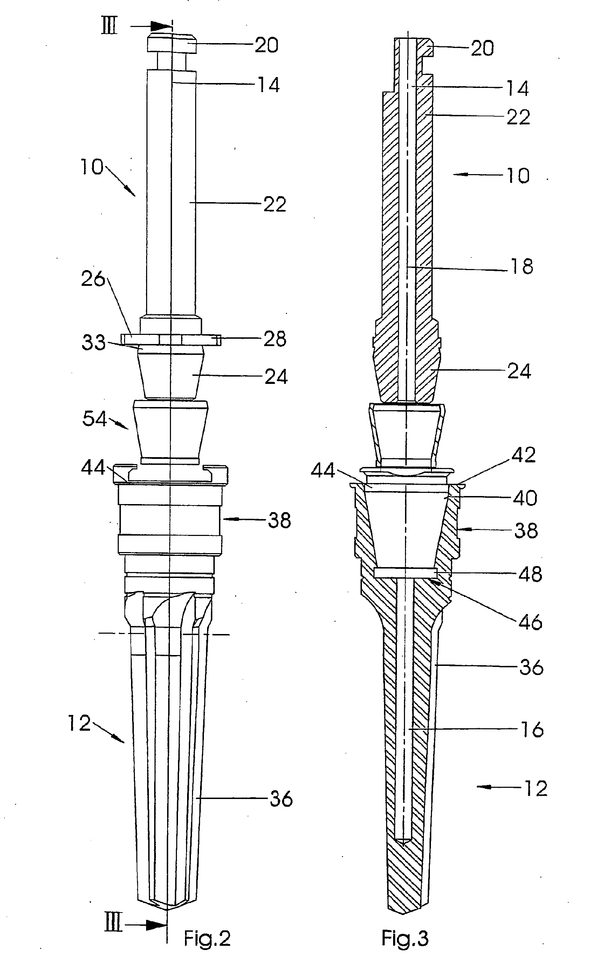

Wie Figur 1 erkennen läßt, weist der erfindungsgemäße Bohrer bei dem dort gezeigten Ausführungsbeispiel ein Halteteil 10 und einen mit dem Halteteile 10 lösbar verbindbaren Bohraufsatz 12 auf, die beide im wesentlichen rotationssymmetrisch zu einer Bohrerachse 14 ausgebildet sind. Der Bohraufsatz 12 und das Halteteil 10 sind, wie aus den Figuren 2 - 5 ersichtlich, von im wesentlichen zentralen, langgestreckten Kühlmittelbohrungen 16, 18 durchsetzt, die im wesentlichen nach Aufbau und Funktion ähnlichen Kühlmittelbohrungen entsprechen, wie sie aus der DE 198 03 998 C2 bekannt sind, auf die insoweit zur Ergänzung der Beschreibung Bezug genommen wird.

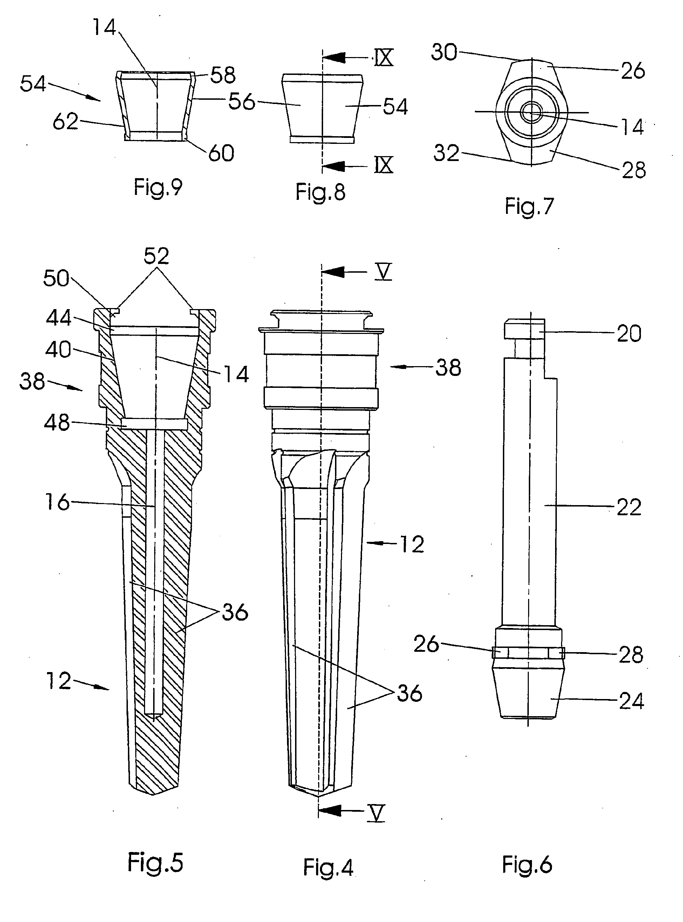

Das Halteteil 10 ist an seinem z. B. in Figur 2 und 3 zeichnerisch oben liegend dargestellten Ende mit einem Kupplungselement 20 versehen, welches in herkömmlicher Weise einen lösbaren Anschluß an einen Bohrerantrieb ermöglicht. An das Kupplungselement 20 schließt ein im wesentlichen hohlzylindrischer Schaft 22 an, der, in Figur 2 und 3 unten gesehen, in einen teilkonusförmig ausgebildeten Schaftkopf 24 übergeht, der sich in Richtung auf das dem Bohraufsatz 12 zugewandte Ende des Halteteiles 10 verjüngt. Im Übergangsbereich zwischen dem Schaft 22 und dem Schaftkopf 24 des Halteteiles 10 springen von der Außenfläche des im Übergangsbereich verdickten Schaftes 22 aus zwei diametral einander gegenüberliegend angeordnete Kopplungsflügel 26, 28 radial nach außen vor, die, wie Figur 7 zeigt, von der Bohrerachse 14 nach außen, in einer zur Bohrerachse 14 senkrechten Ebene gesehen, angeschrägt sind und jeweils in einer entsprechend einer Teilzylinderfläche gekrümmten Umfangsfläche 30, 32 enden. Zwischen dem teilkonusförmigen Schaftkopf 24 und den Kopplungsflügeln 26, 28 weist der Schaft 24 einen an den Schaftkopf 24 anschließenden teilkonusförmigen Einschnappabschnitt auf. Die Kopplungsflügel 26, 28 weisen eine in Figur 7 in der Draufsicht dem Betrachter zugewandten Richtung zur jeweiligen Flügelmitte von den äußeren Schrägflächen ansteigende Oberfläche auf. Zwischen dem Schaft 22 und den Kopplungsflügeln 26, 28 ist am Umfang des Halteteiles 10 ein Außen-Sechskant 34 vorgesehen, der das Ansetzen eines Werkzeuges zwecks Erleichterung der Handhabung des Halteteiles 10 ermöglicht. Das gesamte, vorstehend beschriebene Halteteil ist einstückig aus Metall ausgebildet.

Der Bohraufsatz 12 weist nahe seinem den Bohrschneiden 36 abgewandten Ende eine Hülse 38 auf, deren hohler Innenraum von einer teilkonusförmigen Mantelfläche 40 begrenzt ist, an die nahe einem den Bohrschneiden 36 abgewandten Hülsenrand 42 ein Endabschnitt 44 geringer Höhe (in Axialrichtung gesehen) mit im wesentlichen zylindrischer Innenwand anschließt. Die Steigung der Mantelfläche 40 entspricht im wesentlichen der Steigung des Schaftkopfes 24 des Halteteiles 10.

Nahe einem den Bohrschneiden 36 zugewandten Boden 46 der Hülse 38 weist diese eine im wesentlichen hohlzylindrische, in Axialrichtung im Vergleich zur Länge/Höhe des mit der Mantelfläche 40 versehenen teilkonusförmigen Hülsenabschnittes kurze, im wesentlichen hohlzylindrische Ringerweiterung 48 auf, deren Durchmesser größer ist als derjenige des teilkonusförmigen Abschnittes der Hülse 38 an deren dem Boden 46 naheliegenden Rand.

An den Endabschnitt 44 der Hülse 38 schließt in den Bohrschneiden 36 axial abgewandter Richtung eine im wesentlichen umlaufende Ringkragennut 50 an, die in zwei einander gegenüberliegenden Teilkreisbereichen von einem Stirnringflansch 52 übergriffen wird, der radial in Richtung auf die Bohrerachse 14 vorspringt. Die dem Inneren der Hülse 38 zugewandte Unterfläche des Stirnringflansches 52 verläuft von dem radial innen liegenden Ende des Stirnringflansches 52 in Richtung auf die zylindrischen Innenwandung des Endabschnittes 40 schräg nach, in z. B. Figur 5 gesehen, unten, so daß sich also die axiale Höhe der Ringkragennut 50 von innen nach außen verringert.

Der gesamte Bohraufsatz, wie er vorstehend beschrieben wurde, ist bei dem gezeigten Ausführungsbeispiel einstückig aus Metall ausgebildet.

In die Hülse 38 ist ein Hülseneinsatz 54 einsetzbar, dessen Zentralteil, wie aus Figur 8 und 9 ersichtlich, aus einem Teilkonusabschnitt 56 besteht. Nahe seinem in Figur 8 und 9 oben liegenden Ende schließt an den zentralen, den größten Teil der Axialerstreckung des Hülseneinsatzes 54 bildenden Teilkonusabschnitt ein radial nach innen geneigter Randabschnitt 58 des Hülseneinsatzes 54 an, der radial in Richtung auf die Bohrerachse 14 nach innen geneigt ist. An den Teilkonusabschnitt 56 schließt an der dem Randabschnitt 58 gegenüberliegenden Seite ein einstückig mit dem Teilkonusabschnitt 56 und dem Randabschnitt 58 ausgebildeter Halteabschnitt 60 an, dessen Innenwandung zylindrisch ausgebildet ist und dessen Außenwandung unter Bildung einer Einschnappkerbe 62 leicht nach außen vorspringt.

Die Wandstärke des Hülseneinsatzes 54 ist in Axialrichtung durchgehend im wesentlichen einheitlich. Der Hülseneinsatz 54 ist aus einem viskoseelastischem Kunststoffmaterial hergestellt, dessen Schmelztemperatur so gewählt ist, daß der Kunststoff bei den üblicherweise zum Sterilisieren von Bohrern der erfindungsgemäßen Art verwendeten Sterilisationstemperaturen von mehr als 100°C aufschmilzt oder sich zersetzt.

Der Hülseneinsatz 54 wird werksseitig in die Hülse 38 des Bohraufsatzes 12 hineingedrückt; wobei der Halteabschnitt 60 nach dem Hineindrücken federnd in die Ringerweiterung 48 der Hülse 38 hineinspringt. Angemerkt sei dabei, daß die Neigung, relativ zur Bohrerachse 14, der Mantelfläche 40 des Bohraufsatzes 12 paßgenau mit der Neigung des Teilkonusabschnittes 56 des Hülseneinsatzes 54 und auch der teilkonusförmigen Außenfläche des Schaftkopfes 24 im wesentlichen übereinstimmt.

In der zahnärztlichen Praxis wird der mit dem Hülseneinsatz 54 versehene Bohraufsatz 12 auf das Halteteil 10 in einer derartigen relativen Drehwinkelstellung aufgedrückt, daß die Kopplungsflügel 26, 28 in von dem Stirnringflansch 52 freigelassene Ringkragenfenster 64, 66 des Stirnringflansches 52 eingreifen können. Bei Ausübung von leichtem Druck wird einerseits durch den Schaftkopf 24 das Kunststoffmaterial des Endabschnittes 60 des Hülseneinsatzes 54 noch weiter in die Ringerweiterung 48 der Hülse 38 hineingedrückt. Zusätzlich schnappt der Randabschnitt 58 des Hülseneinsatzes 54 übergreifend über den Einschnappabschnitt des Halteteiles 10, wodurch das Halteteil 10 und der Bohraufsatz 12 bereits in Axialrichtung relativ gegeneinander gesichert werden. Bei Beginn der Rotation des Halteteiles 22, in welcher Drehrichtung auch immer, drehen sich die Kopplungsflügel 26, 28 des Halteteiles 10 aus den Ringkragenfenstern 64, 66 in die Ringkragennut 50 hinein, wobei sich infolge der sich in Richtung auf die Flügelmitte verdickenden Form der Kopplungsflügel eine selbsthemmende Form- und Kraftschlußverbindung zwischen den Kopplungsflügeln 26, 28 und dem Stirnringflansch 52 ergibt.

Hierdurch wird eine automatische Verbindung lösbarer Art zwischen dem Halteteil 10 und dem Bohraufsatz 12 erzielt, die sowohl in axialer als auch in Verdrehrichtung zuverlässig ist, sich aber bei Umkehren der Drehbewegung ohne weiteres lösen läßt, sobald die Kopplungsflügel 26, 28 wieder in die Ringkragenfenster 64, 66 hineingedreht werden; in der letztgenannten Position läßt sich der Bohraufsatz 12 leicht wieder vom Halteteil 10 abziehen.

Der erfindungsgemäße Bohraufsatz 12 ist nur einmal verwendbar, und zwar aus folgendem Grund: Wenn der Bohraufsatz 12 vom Halteteiles 10 abgezogen worden ist, muß er weggeworfen werden. Tut der Zahnarzt dies nicht, sondern versucht, entgegen der Gebrauchsanweisung, sozusagen, den Bohraufsatz 12 zu sterilisieren, so zersetzt sich der Hülseneinsatz 54, woraufhin ein erneutes Verbinden des Bohraufsatzes 12 mit dem Halteteil 10 nicht mehr möglich wird, da der Schaftkopf 24 in der Hülse 38 keinen Halt mehr findet.

## Patentansprüche

1. Bohrer zur Herstellung einer Bohrung in einem Knochengewebe, mit einer Kupplungseinrichtung, einem als Halteteil ausgebildeten ersten Teil und einem als Bohraufsatz ausgebildeten zweiten Teil, welcher mit dem Halteteil über die Kupplungseinrichtung lösbar verbunden werden kann,
wobei die Kupplungseinrichtung einen im wesentlichen zur Bohrerachse rotationssymmetrischen Schaft an einem der beiden Teile und eine im wesentlichen zur Bohrerachse rotationssymmetrische Hülse (38) mit einer zu dem Schaft komplementären Zentralbohrung an dem anderen der beiden Teile, in welche der Schaft einführbar ist, und weiterhin eine Sicherungseinrichtung zum Verhindern einer Relativbewegung in Rotations- und Axialrichtung zwischen dem Halteteil und dem Bohraufsatz aufweist,
derart, dass im eingekuppelten Zustand der Schaft in die Zentralbohrung eingreift und der Bohraufsatz an dem Halteteil gesichert ist; und
wobei zumindest ein Rastelement an einem der beiden Teile und eine zugehörige Aussparung an dem anderen der beiden Teile zum formschlüssigen Verbinden und Sichern des Halteteiles und des Bohraufsatzes gegen eine Relativbewegung in Längsrichtung vorgesehen sind,
die Sicherungseinrichtung zumindest ein Sicherungselement (54) aufweist, welches ein Sterilisieren des Bohraufsatzes (12) und damit dessen mehrmalige Verwendung **dadurch** verhindert, dass es aufschmilzt oder sich zersetzt,
die Hülse (38) am Bohraufsatz (12) und der Schaft (22, 24) am Halteteil (10) ausgebildet sind;
das Sicherungselement einen in die zum Aufnehmen des Schaftes (22, 24) dienende Zentralbohrung der Hülse (38) einsetzbaren Hülseneinsatz (54) aus einem unter Sterilisationsbedingungen sich irreversibel in einem eine Wiederverwendung des Bohraufsatzes (12) ausschließenden Ausmaß verändernden Material aufweist, in deren Innenbohrung der Schaft (22, 24) im wesentlichen passgenau eingreifen kann, **dadurch gekennzeichnet, dass**
der Hülseneinsatz (54) aus bei Sterilisationstemperatur sich zersetzendem und/oder aufschmelzendem Kunststoff besteht,
der Hülseneinsatz (54) einen Teilkonusabschnitt (56) mit teilkonusförmiger Außen-und Innen-Mantelfläche, beide im wesentlichen parallel zueinander verlaufend, aufweist;
der Schaft (22) in seinem in den Hülseneinsatz (54) einführbaren Bereich einen Schaftkopf (24) mit Teilkonusform aufweist, dessen Außenmantelfläche komplementär zur Innenfläche des Hülseneinsatzes (54) in dessen Teilkonusabschnitt (56) ausgebildet ist;
die Hülse (38) im zur Aufnahme des Hülseneinsatzes (54) bestimmten, Bohrschneiden (36) des Bohraufsatzes (12) abgewandten Endbereich eine teilkonusförmige Innenbohrung aufweist, in die der Hülseneinsatz (54) einsetzbar ist,
nahe einem den Bohrschneiden (36) zugewandten Boden (46) des zur Aufnahme des Hülseneinsatzes (54) dienenden Endabschnittes (44) der Hülse (38) eine radial nach außen vorspringende Ringerweiterung (48) vorgesehen ist;
der Hülseneinsatz (54) nahe seinem dem Boden (46) der Hülse (38) zugewandten Ende einen im wesentlichen hohlzylindrischen, jedoch leicht nach aussen vorspringenden Halteabschnitt (60) aufweist; und
nahe dem dem Halteabschnitt (60) des Hülseneinsatzes (54) abgewandten Rand des Hülseneinsatzes (54) ein Randabschnitt (58) anschliesst, der sich in Axialrichtung vom weiten Ende des Teilkonusabschnittes (56) in Richtung auf die Bohrerachse (14) nach innen neigt.

2. Bohrer nach Anspruch 1, **dadurch gekennzeichnet, dass** die Sicherungseinrichtung eine nahe dem dem Schaft (22) zugewandten Stirnende des Bohraufsatzes (12) angeordnete Ringkragennut (40) mit radial nach innen vorspringendem Stirnringflansch (52) aufweist, in den mindestens zwei in Umfangsrichtung langgestreckte Ringkragenfenster (64, 66) eingeschnitten sind, und
dass der Schaft in einem an den Schaftkopf (24) anschliessenden Sicherungsbereich radial nach aussen vorspringende Kopplungsflügel (26, 28) aufweist, die komplementär zu en Ringkragenfenstern (64, 66) der Hülse (38) ausgebildet sind, deren axiale Höhe geringfügig kleiner ist als die axiale Höhe, jeweils in Richtung der Bohrachse (14) gesehen, der Ringkragennut (50), und deren Umfangserstreckung kleiner ist als die Umfangserstreckung der Ringkragenfenster (64, 66).

3. Bohrer nach Anspruch 2, **dadurch gekennzeichnet, dass** die Kopplungsflügel (26, 28) zu ihren radial äußeren Enden hin in einer senkrecht zur Bohrerachse (14) liegenden Ebene angeschrägt sind;
dass die radial außen liegenden Umfangsflächen (30, 32) der Kopplungsflügel (26, 28) entsprechend der radial liegenden äußeren Innenwandung der Ringkragennut (50) gekrümmt sind; und
dass die Dicke der Flügel von den schrägen Aussenkanten zur jeweiligen Flügelmitte hin ansteigt.

4. Bohrer nach Anspruch 3, **dadurch gekennzeichnet, dass** die dem Schaft (22) abgewandte radiale Dachfläche der Ringkragennut (40) von den Ringkragenfenstern (64, 66) her unter Verringerung der axialen Höhe der Ringkragennut (50) auf die jeweilige Mitte des betreffenden Stirnringflanschsegmentes (52) hin abnimmt.

## Claims

1. Trephine for creating a borehole in bone tissue, with a coupling device, a first part designed as a holding part and a second part designed as a drill attachment, the latter being detachably connected to the holding part by means of the coupling device,
wherein the coupling device has a shaft that is essentially rotationally symmetrical to the drill axis on one of the two parts, and a sleeve (38) that is essentially rotationally symmetrical to the drill axis with a central bore hole complementary to the shaft on the other of the two parts, into which the shaft can be inserted, and furthermore a safety device to prevent a relative movement in the rotational and axial directions between the holding part and the drill attachment,
such that in the coupled state, the shaft engages in the central borehole and the drill attachment is secured on the holding part; and
wherein at least one locking element is provided on one of the two parts and a corresponding recess is provided on the other of the two parts for the positive-fitting connection and securing of the holding part and the drill attachment to prevent a relative movement in the longitudinal direction,
the safety device has at least one safety element (54) which prevents sterilisation of the drill attachment (12) and thus any repeat use, in that it melts or decomposes,
the sleeve (38) is formed on the drill attachment (12) and the shaft (22, 24) is formed on the holding part (10);
the safety element has a sleeve insert (54) which can be deployed in the central borehole of the sleeve (38) to accommodate the shaft (22, 24) and which is of a material that can change irreversibly under sterilisation conditions to such an extent that re-use of the drill attachment (12) is excluded, and into the inner bore of which the shaft (22, 24) can essentially engage in an exact fit, **characterised in that**
the sleeve insert (54) comprises synthetic material that breaks down and/or melts at sterilisation temperature,
the sleeve insert (54) has a partial conical section (56) with an outer and inner casing surface in the shape of a partial cone, both running essentially parallel to one another;
the shaft (22) has, in its area that can be inserted into the sleeve insert (54), a shaft head (24) of a partial conical shape, whose outer casing surface is designed to be complementary to the inner surface of the sleeve insert (54) in its partial conical section (56);
the sleeve (38) has, in the end area intended for accommodating the sleeve insert (54) and facing away from the drill bits (36) of the drill attachment (12), a partially conical inner borehole, into which the sleeve insert (54) can be inserted,
a ring extension (48) which projects radially outwards is provided close to a base (46), which faces the drill bits (36), of the end section (44) of the sleeve (38), which serves to accommodate the sleeve insert (54);
the sleeve insert (54) has, close to its end facing the base (46) of the sleeve (38), an essentially hollow cylindrical holding section (60) which however projects slightly outwards; and
following on close to the edge of the sleeve insert (54) that faces away from the holding section (60) of the sleeve insert (54) is an edge section (58) which inclines inwards in an axial direction from the wide end of the partial conical section (56) in the direction towards the drill axis (14).

2. Trephine according to claim 1, **characterised in that** the safety device has a ring collar groove (40), arranged close to the end face of the drill attachment (12) that faces the shaft (22), with an end face ring flange (52) which projects radially inwards, cut into which are at least two ring collar windows (64, 66) which extend in the circumferential direction, and
that the shaft, in a securing area following on from the shaft head (24), has coupling wings (26, 28) which project radially outwards and which are designed to be complementary to the ring collar windows (64, 66) of the sleeve (38), whose axial height is slightly smaller than the axial height, viewed respectively in the direction of the drill axis (14), of the ring collar groove (50), and whose circumferential extension is smaller than the circumferential extension of the ring collar windows (64, 66).

3. Trephine according to claim 2, **characterised in that** the coupling wings (26, 28) are bevelled towards their radially outer ends, in a plane lying perpendicular to the drill axis (14);
that the radially outer circumferential surfaces (30, 32) of the coupling wings (26, 28) are curved in accordance with the radially inner walling of the ring collar groove (50); and
that the thickness of the wings increases from the oblique outer edges towards the respective wing centre.

4. Trephine according to claim 3, **characterised in that** the radial roof surface of the ring collar groove (40), facing away from the shaft (22), decreases from the collar groove (40) of the ring collar windows (64, 66) towards the respective centre of the corresponding end face ring flange segment (52), with a reduction in the axial height of the ring collar groove (50).

## Revendications

1. Foret destiné à réaliser un perçage dans un tissu osseux, avec un dispositif d'accouplement, une première partie, réalisée sous forme de pièce de retenue, et une deuxième partie, réalisée sous forme de mèche, pouvant être reliée, de manière désolidarisable, à la pièce de retenue par l'intermédiaire du dispositif d'accouplement,
le dispositif d'accouplement présentant une queue, répondant sensiblement à une symétrie de rotation par rapport à l'axe de foret, sur l'une des deux parties et une douille (38), répondant sensiblement à une symétrie de rotation par rapport à l'axe de foret, avec un perçage central, complémentaire à la queue, sur l'autre des deux parties, dans lequel la queue est susceptible d'être introduite, et présentant en outre un dispositif de sécurité pour empêcher tout déplacement relatif en direction de rotation et axiale, entre la pièce de retenue et le foret,
de manière que, à l'état introduit et accouplé, la queue s'engage dans le perçage central et que le foret soit assuré sur la pièce de retenue ; et
au moins un élément d'encliquetage étant prévu sur au moins l'une des deux parties et un évidement afférent étant prévu sur l'autre des deux parties, pour assurer une liaison par ajustement de forme et assurer la pièce de retenue et le foret contre tout déplacement relatif en direction longitudinale,
le dispositif de sécurité présentant au moins un élément de sécurité (54), empêchant, par le fait qu'il fond ou se désagrège, une stérilisation du foret (12) et, ainsi, sa réutilisation répétée,
la douille (38) étant réalisée sur le foret (12) et la queue (22, 24) étant réalisée sur la pièce de retenue (10) ;
l'élément de sécurité présentant un insert de douille (54) susceptible d'être inséré dans le perçage central, servant à recevoir la queue (22, 24), de la douille (38), l'insert de douille étant composé d'un matériau changeant de dimensions de manière irréversible dans des conditions de stérilisation, en excluant toute réutilisation du foret (12), insert de douille (54) dans le perçage intérieur duquel la queue (22, 24) peut s'engager sensiblement avec une exactitude de repérage, **caractérisé en ce que**
l'insert de douille (54) est composé d'une matière synthétique se désagrégeant et/ou fondant à la température de stérilisation,
l'insert de douille (54) présente un tronçon en cône partiel (56), avec une surface d'enveloppe extérieure et une surface d'enveloppe intérieure en forme de cône partiel, les deux s'étendant sensiblement parallèlement entre elles,
la queue (22) présente, dans sa zone susceptible d'être introduite dans l'insert de douille (54), une tête de queue (24) en forme de cône partiel, dont la surface d'enveloppe extérieure est de configuration complémentaire à la surface intérieure de l'insert de douille (54) dans son tronçon en cône partiel (56) ;
la douille (38) présente, dans une zone d'extrémité, déterminée pour recevoir l'insert de douille (54), opposée à des tranchants de perçage (36) du foret (12), un perçage intérieur en forme de cône partiel, dans lequel l'insert de douille (54) est susceptible d'être inséré ;
un élargissement annulaire (48), se projetant radialement vers l'extérieur, est prévu à proximité d'un fond (46), tourné vers les tranchants de perçage (36), du tronçon d'extrémité (44), servant à recevoir l'insert de douille (54), de la douille (38) ;
l'insert de douille (54) présente, à proximité de son extrémité tournée vers le fond (46) de la douille (38), un tronçon de retenue (60) sensiblement cylindrique et creux, faisant cependant légèrement saillie vers l'extérieur ; et
un tronçon de bordure (58), incliné vers l'intérieur en direction de l'axe de foret (14), en direction axiale en partant de l'extrémité large du tronçon en cône partiel (56), se raccorde à proximité du bord, opposé au tronçon de retenue (60) de l'insert de douille (54), de l'insert de douille (54).

2. Foret selon la revendication 1, **caractérisé en ce que** le dispositif de sécurité présente une gorge de collerette annulaire (40), disposée à proximité de l'extrémité frontale, tournée vers la queue (22), du foret (12), avec un bride annulaire frontale (52) faisant saillie radialement vers l'intérieur, dans laquelle sont taillées au moins deux fenêtres de collerette annulaire (64, 66) allongées en direction périphérique, et
**en ce que** la queue présente, dans une zone de sécurité se raccordant à la tête de queue (24), des ailettes d'accouplement (26, 28) faisant saillie radialement vers l'extérieur, conformées de manière complémentaire aux fenêtres de collerette annulaire (64, 66) de la douille (38), dont la hauteur axiale est légèrement inférieure à la hauteur axiale, chaque fois observée en direction de l'axe de perçage (14), de la gorge de collerette annulaire (50), et dont l'étendue périphérique est plus petite que l'étendue périphérique des fenêtres de collerette annulaire (64, 66).

3. Foret selon la revendication 2, **caractérisé en ce que** les ailettes d'accouplement (26, 28) sont chanfreinées à leurs extrémités radialement extérieures dans un plan disposé perpendiculairement à l'axe de perçage (14) ;
**en ce que** les surfaces périphériques (30, 32), situées radialement extérieurement, des ailettes d'accouplement (26, 28) sont incurvées de manière correspondante à la paroi intérieure située radialement extérieurement de la gorge de collerette annulaire (50) ; et
**en ce que** l'épaisseur des ailettes augmente en allant des arêtes extérieures obliques vers le centre d'ailette respectif.

4. Foret selon la revendication 3, **caractérisé en ce que** la surface en toit radiale, opposée à la queue (22), de la gorge de collerette annulaire (40) va en diminuant, en partant des fenêtres de collerette annulaire (64, 66), avec diminution de la hauteur axiale de la gorge de collerette annulaire (50), jusqu'au centre respectif du segment de bride annulaire frontale (52) concerné.
